(19) Europäisches Patentamt
European Patent Office
Office européen des brevets

(11) Veröffentlichungsnummer: **0 385 267 A2**

(12) **EUROPÄISCHE PATENTANMELDUNG**

(21) Anmeldenummer: **90103432.2**

(22) Anmeldetag: **22.02.90**

(51) Int. Cl.⁵: **C07D 213/30, C07D 213/26, C07D 213/89, A01N 43/40**

(30) Priorität: **01.03.89 CH 747/89**

(43) Veröffentlichungstag der Anmeldung: **05.09.90 Patentblatt 90/36**

(84) Benannte Vertragsstaaten: **AT BE CH DE ES FR GB GR IT LI NL**

(71) Anmelder: **F. HOFFMANN-LA ROCHE AG**
**Postfach 3255**
**CH-4002 Basel(CH)**

(72) Erfinder: **Isenring, Hans Peter Dr.**
**Himmelrainweg 5**
**CH-4450 Sissach(CH)**
Erfinder: **Zehnder, Beat, Dr.**
**Brunngasse 89**
**CH-4153 Reinach(CH)**
Erfinder: **Ziegler, Hugo, Dr.**
**Oberwilerstrasse 39**
**CH-4123 Allschwil(CH)**

(74) Vertreter: **Urech, Peter, Dr. et al**
**Grenzacherstrasse 124 Postfach 3255**
**CH-4002 Basel(CH)**

(54) **Pyridinderivate, deren Herstellung und Verwendung als Fungizide.**

(57) Die Erfindung betrifft neue Pyridinderivate der Formel

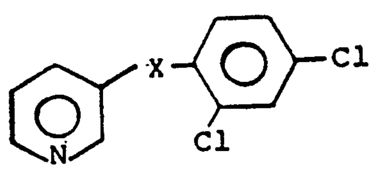

I

worin X eine Gruppe

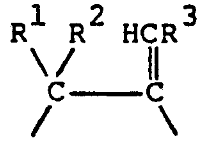

(a) oder

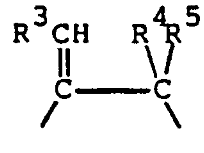

(b)

bedeutet und $R^1$, $R^2$, $R^3$, $R^4$ und $R^5$ die in der Beschreibung angegebenen Bedeutungen besitzen, und N-Oxide, N-Amino-Salze, Kupfer-Komplexe und Säureadditionssalze davon, Verfahren zur Herstellung dieser Substanzen, fungizide Mittel, die diese Substanzen als Wirkstoff enthalten, sowie die Verwendung solcher Substanzen bzw. Mittel zur Bekämpfung von Fungi in der Landwirtschaft und im Gartenbau.

EP 0 385 267 A2

## Pyridinderivate, deren Herstellung und Verwendung als Fungizide

Die vorliegende Erfindung betrifft heterocyclische Verbindungen, und zwar Pyridinderivate der allgemeinen Formel

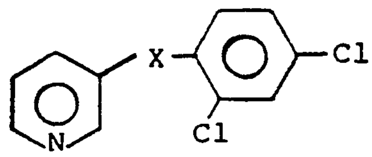

I

worin
X eine Gruppe (a) oder (b)

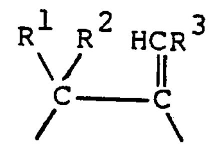

(a)

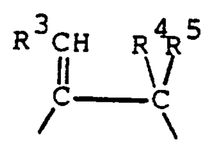

(b)

$R^1$ und $R^2$ Methyl oder zusammen Aethylen,
$R^3$ Wasserstoff, Chlor, $C_{1-4}$-Alkyl oder Methoxy,
$R^4$ Wasserstoff oder Methyl
und
$R^5$ Methyl oder, falls $R^4$ Wasserstoff bedeutet, auch $C_{1-4}$-Alkyl, Allyl oder Propargyl,
oder
$R^4$ und $R^5$ zusammen Aethylen bedeuten,
sowie die N-Oxide, N-Amino-Salze und Kupfer-Komplexe der Verbindungen der Formel I, und schliesslich die Säureadditionssalze der Verbindungen der Formel I und ihrer N-Oxide.

Alle diese erfindungsgemässen Verbindungen besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der erfindungsgemässen Verbindungen, fungizide Mittel, die solche Verbindungen als Wirkstoffe enthalten, sowie die Verwendung solcher Verbindungen und Mittel zur Bekämpfung von Fungi in der Landwirtschaft und im Gartenbau.

Unter dem Ausdruck "N-Amino-Salze" sind die Verbindungen der Formel I zu verstehen, deren 3-Pyridylgruppe gemäss der nachfolgenden Formel modifiziert ist:

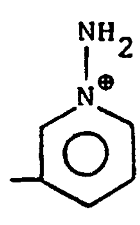

$(A^1)^\ominus$

worin $(A^1)^\ominus$ das Anion einer physiologisch verträglichen Säure bedeutet.
Beispiele solcher Säuren sind Alkancarbonsäuren, Benzoesäure und deren kernsubstituierte Derivate, wie beispielsweise alkyl-, nitro- und/oder chlorsubstituierte Benzoesäuren, gegebenenfalls substituierte Benzolsulfonsäure; Carbamin säure, Salzsäure, Bromwasserstoffsäure, Jodwasserstoffsäure und Schwefelsäure.

Als Säureadditionssalze der Verbindungen der Formel I kommen physiologisch verträgliche Salze in

Frage. Hierzu gehören vorzugsweise Salze dieser Verbindungen mit anorganischen und organischen Säuren, wie Salzsäure; Salpetersäure; Phosphorsäure; mono- und bifunktionellen Carbonsäuren und Hydroxycarbonsäuren, z.B. Essigsäure, Maleinsäure, Bernsteinsäure, Fumarsäure, Weinsäure, Zitronensäure, Salicylsäure, Sorbinsäure und Milchsäure; und Sulfonsäuren, z.B. 1,5-Naphthalin-disulfonsäure.

Auch die N-Oxide der Verbindungen der Formel I können mit geeigneten Säuren Säureadditionssalze bilden. Es kommen als derartige Säureadditionssalze physiologisch verträgliche Salze, vorzugsweise Salze mit starken Säuren in Frage, wie anorganischen Säuren, z.B. Salzsäure, Salpetersäure und Phosphorsäure, und Sulfonsäuren, z.B. 1,5-Naphthalin-disulfonsäure.

Mit der Bezeichnung "Kupfer-Komplexe" sind insbesondere Kupfer(II)-Komplexe der Formel

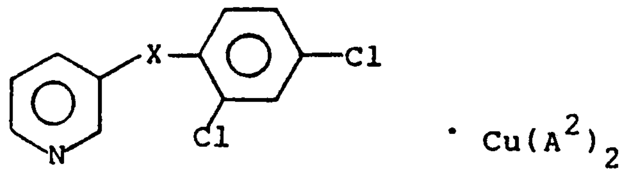

gemeint, wobei $A^2$ das Anion einer organischen oder anorganischen Säure, wie Ameisensäure, Essigsäure, Propionsäure, Benzoesäure, Oxalsäure, Weinsäure, Milchsäure, Salicylsäure, Zitronensäure, Kohlensäure, Schwefelsäure, Salpetersäure oder Salzsäure, bedeutet. Vorzugsweise bedeutet $A^2$ Acetat.

Die Verbindungen können als E- und Z-Isomere und/oder als Atropisomere vorliegen. Die Formel I soll all diese möglichen isomeren Formen und deren Gemische umfassen.

In der Formel I bedeuten $R^1$ und $R^2$ vorzugsweise zusammen Aethylen. Unabhängig von den Bedeutungen von $R^1$ und $R^2$ und voneinander bedeuten $R^3$ vorzugsweise Wasserstoff oder Methoxy, $R^4$ vorzugsweise Wasserstoff und $R^5$ vorzugsweise Methyl.

Besonders bevorzugte einzelne erfindungsgemässe Verbindungen sind:
3-(2,4-Dichlor-α,α-dimethyl-β-methylenphenäthyl)-pyridin,
3-[1-(2,4-Dichlor-α-methylenbenzyl)-cyclopropyl]-pyridin,
1:1 Komplex von 3-[1-(2,4-Dichlor-α-methylenbenzyl)-cyclopropyl]-pyridin und Kupfer(II)-acetat,
3-[1-(2,4-Dichlor-α-methylenbenzyl)cyclopropyl]-pyridin-1-oxid,
3-[1-(2,4-Dichlor-α-methoxymethylenbenzyl)-cyclopropyl]-pyridin,
3-[1-(2,4-Dichlor-α-äthylidenbenzyl)-cyclopropyl]-pyridin,
3-(2,4-Dichlor-α-methoxymethylen-β-methylphenäthyl)-pyridin,
3-(α-Butyliden-2,4-dichlor-β-methylphenäthyl)-pyridin,
3-(2,4-Dichlor-β,β-dimethyl-α-methylenphenäthyl)-pyridin,
3-[2,4-Dichlor-α-methylen-β-(2-propinyl)-phenäthyl]-pyridin,
3-[2,4-Dichlor-α-methoxymethylen-β-(2-propinyl)-phenäthyl]-pyridin,
3-(2,4-Dichlor-α-methylen-β-propylphenäthyl)-pyridin,
3-(α-Chlormethylen-2,4-dichlor-β-methylphenäthyl)-pyridin,
3-[α-Chlormethylen-2,4-dichlor-β-(2-propinyl)-phenäthyl]-pyridin,
3-(β-Allyl-2,4-dichlor-α-methylenphenäthyl)-pyridin,
1:1-Komplex von 3-(α-Butyliden-2,4-dichlor-β-methylphenäthyl)-pyridin und Kupfer(II)-acetat,
3-[α-Butyliden-2,4-dichlor-β-(2-propinyl)-phenäthyl]-pyridin,
3-(2,4-Dichlor-β-methyl-α-methylenphenäthyl)-pyridin,
1:1-Komplex von 3-(2,4-Dichlor-β-methyl-α-methylenphenäthyl)-pyridin und Kupfer(II)-acetat und
3-(2,4-Dichlor-β-äthyl-α-methoxymethylenphenäthyl)-pyridin,
insbesondere die zweitgenannte Verbindung obiger Liste.

Weitere Vertreter der erfindungsgemässen Verbindungen sind:
3-(2,4-Dichlor-α-äthyliden-β-methylphenäthyl)-pyridin,
1:1-Komplex von 3-(2,4-Dichlor-β,β-dimethyl-α-methylenphenäthyl)-pyridin und Kupfer(II)-acetat,
1:1-Komplex von 3-(α-Butyliden-2,4-dichlor-β-methylphenäthyl)-pyridin und Kupfer(II)-acetat,
3-(2,4-Dichlor-β-methyl-α-propylidenphenäthyl)-pyridin,
1:1-Komplex von 3-(2,4-Dichlor-β-methyl-α-propylidenphenäthyl)-pyridin und Kupfer(II)-acetat,
1:1-Komplex von 3-[2,4-Dichlor-α-methoxymethylen-β-(2-propinyl)-phenäthyl]-pyridin und Kupfer(II)-acetat,
3-(2,4-Dichlor-β-methyl-α-methylenphenäthyl-pyridin-1-oxid,
1-Amino-3-(2,4-dichlor-β-methyl-α-methylenphenäthyl)-pyridinium-2,4,6-trimethylbenzolsulfonat und
1-Amino-3-[1-(2,4-dichlor-α-methylenbenzyl)-cyclopropyl]-pyridinium-2,4,6-trimethylbenzolsulfonat.

Das erfindungsgemässe Verfahren zur Herstellung der erfindungsgemässen Verbindungen ist dadurch

gekennzeichnet, dass man

a) ein Keton der allgemeinen Formel

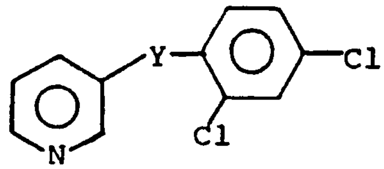

II

worin Y eine Gruppe (a′) oder (b′)

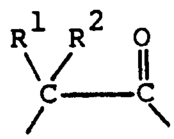

(a′)

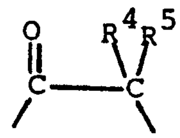

(b′)

und $R^1$, $R^2$, $R^4$ und $R^5$ die oben angegebenen Bedeutungen besitzen, mit einem Triphenylphosphoniumsalz der allgemeinen Formel

$(C_6H_5)_3P^{\oplus}CH_2R^3Hal^{\ominus}$ III

worin $R^3$ die oben angegebene Bedeutung besitzt und $Hal^{\ominus}$ ein Chlor- oder Bromion bedeutet, umsetzt,

b) zwecks Herstellung des N-Oxids oder eines N-Amino-Salzes einer Verbindung der Formel I diese N-oxidiert bzw. mit einem Aminderivat der allgemeinen Formel

$H_2N\text{-}A^3$ IV

worin $A^3$ den Rest einer physiologisch verträglichen Säure bedeutet, umsetzt,

c) zwecks Herstellung eines Säureadditionssalzes einer Verbindung der Formel I oder deren N-Oxids die Verbindung der Formel I bzw. deren N-Oxid mit einer Säure umsetzt, oder

d) zwecks Herstellung eines Kupfer-Komplexes einer Verbindung der Formel I diese mit einem Kupfersalz der allgemeinen Formel

$Cu(A^2)_2$ V

worin $A^2$ die oben angegebene Bedeutung besitzt, umsetzt.

Die Verfahrensvariante a) wird zweckmässigerweise durchgeführt, indem man ein Triphenylphosphoniumsalz der Formel III in einem inerten Verdünnungsmittel, wie einem aromatischen Kohlenwasserstoff, z.B. Benzol oder Toluol, einem aliphatischen oder cyclischen Aether, z.B. 1,2-Dimethoxyäthan, Tetrahydrofuran oder Dioxan, oder einem Dialkylamid, z.B. Dimethylformamid, suspendiert und mit einer Base, wie einem Alkalimetallhydrid, z.B. Natriumhydrid, einem Alkalimetallalkoholat, z.B. Natriummethylat oder Kalium-tert.butylat, einem Alkalimetallamid, z.B. Natriumamid, oder einem Lithiumalkyl, z.B. n-Butyllithium, bei Temperaturen zwischen -20 °C und der Rückflusstemperatur des Reaktionsgemisches, vorzugsweise im Temperaturbereich von 0 °C bis 80 °C, reagieren lässt. Danach wird ein Keton der Formel II, ungelöst oder zweckmässigerweise in einem der oben erwähnten Verdünnungsmittel gelöst, zugegeben und das Reaktionsgemisch im Temperaturbereich von -75 °C bis zur Rückflusstemperatur, vorzugsweise zwischen +20 °C und 80 °C, gehalten. Als Triphenylphosphoniumsalz und Base eignen sich ganz besonders die als "Instant-Ylide" (Fluka) käuflichen Gemische eines Triphenylphosphoniumsalzes mit Natriumamid.

Die N-Oxid-Bildung nach Verfahrensvariante b) erfolgt zweckmässigerweise unter denjenigen Reaktionsbedingungen, die in der Europäischen Patentschrift Nr. 74018, Seite 5, Zeilen 47-61, für die N-Oxidation von Pyridin- und Pyrazinderivate beschrieben sind.

Die N-Aminierung einer Verbindung der Formel I mit einem Aminderivat der Formel IV, also die Verfahrensvariante b), wird zweckmässigerweise so durchgeführt, dass man die Verbindung I mit dem

Aminderivat in einem inerten organischen Verdünnungsmittel, wie einem halogenierten aliphatischen Kohlenwasserstoff, z.B. Methylenchlorid oder Chloroform, bei Temperaturen zwischen 0° C und der Rückflusstemperatur des Reaktionsgemisches behandelt.

Zur Herstellung der Säureadditionssalze werden die Verbindungen I bzw. deren N-Oxide mit den gewünschten Säuren auf übliche Weise umgesetzt.

Die Verfahrensvariante d) wird zweckmässigerweise so durchgeführt, dass man eine Lösung der Verbindung der Formel I in einem inerten, mit Wasser nicht mischbaren Verdünnungsmittel, wie einem halogenierten aliphatischen Kohlenstoff, z.B. Methylenchlorid oder Chloroform, und eine wässrige Lösung des Kupfersalzes der Formel V miteinander intensiv rührt.

Die Isolierung und Reinigung der so hergestellten Verbindungen der Formel I und deren N-Oxide, N-Amino-Salze, Säureadditionssalze und Kupfer-Komplexe kann nach an sich bekannten Methoden erfolgen. Ebenfalls nach an sich bekannten Methoden können allfällig erhaltene Isomerengemische, z.B. E/Z-Isomerengemische, in die reinen Isomeren aufgetrennt werden, beispielsweise durch Chromatographie oder fraktionierte Kristallisation.

Die als Ausgangsmaterialien in der Verfahrensvariante a) verwendeten Ketone der allgemeinen Formel II sind ent weder bekannt oder können nach an sich bekannten Methoden hergestellt werden [siehe z.B. die Europäischen Patentpublikationen Nrn. 74018 und 117485 sowie - im Falle der Herstellung der Cyclopropylketone der Formel II ($R^1$ und $R^2$ bzw. $R^4$ und $R^5$ bedeuten zusammen Aethylen) - Tetrahedron Letters, 25, 5501 (1984)]. Ebenfalls bekannt und nach an sich bekannten Methoden herstellbar sind die Reagentien der Formeln III und IV.

Die erfindungsgemässen Verbindungen besitzen fungizide Wirkung und können dementsprechend zur Bekämpfung von Pilzen in der Landwirtschaft, im Gartenbau sowie in der Holzverarbeitung Verwendung finden. Sie eignen sich insbesondere zur Hemmung des Wachstums oder zur Vernichtung von phytopathogenen Pilzen auf Pflanzenteilen, z.B. Blättern, Stengeln, Wurzeln, Knollen, Früchten oder Blüten, und auf Saatgut sowie im Erdboden. Ferner können mit den erfindungsgemässen Verbindungen holzabbauende und holzverfärbende Pilze bekämpft werden. Die erfindungsgemässen Verbindungen sind besonders wirksam bei der Bekämpfung von Pilzen der Klassen Deuteromycetes, Ascomycetes und Basidiomycetes, wie beispielsweise Botrytis cinerea, Erysiphe cichoracearum, Erysiphe graminis, Uncinula necator, Podosphaera leucotricha, Venturia inaequalis, Cercospora archidicola, Cercospora beticola und Mycosphaerella fijiensis sowie von Schadpilzen der Gattungen Sphaerotheca, Puccinia, Uromyces, Hemileia, Rhizoctonia, Alternaria, Cercosporella, Ceratocystis (z.B. Ceratocystis ulmi und Ceratocystis fimbriata), Verticillium, Fusarium, Helmithosporium, Sclerotinia, Penicillium, Septona, Ustilago, Tilletia, Coniophora, Gloeophyllum und Aureobasidium.

Die erfindungsgemässen Verbindungen zeichnen sich durch lokale und bzw. oder systemische Wirkung aus.

Die erfindungsgemässen Verbindungen wirken gegen phytopathogene Pilze unter Gewächshausbedingungen bereits bei Konzentrationen von 1 mg bis 500 mg Wirkstoff pro Liter Spritzbrühe. Im Freiland werden vorteilhaft Konzentrationen von 25 g bis 1500 g Wirkstoff der Formel I pro Hektar und Behandlung zur Anwendung gebracht. Zur Bekämpfung von samen- oder bodenbürtigen Pilzen im Beizverfahren werden mit Vorteil 0,05 g bis 1,5 g Wirkstoff der Formel I pro kg Samen verwendet.

Auch die oben erwähnten Ausgangsmaterialien der allgemeinen Formel II sind als Fungizide wertvoll, da sie ein ähnliches Aktivitätsspektrum wie die Verbindungen der Formel I aufweisen. Diese Materialien können dementsprechend auch zur Bekämpfung von Fungi in der Landwirtschaft und im Gartenbau eingesetzt werden, und zwar auf gleiche Weise wie die erfindungsgemässen Verbindungen.

Die erfindungsgemässen Verbindungen können zu verschiedenartigen Mitteln, z.B. Lösungen, Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame Menge mindestens einer Verbindung der allgemeinen Formel I, wie oben definiert, oder eines N-Oxids, N-Amino-Salzes, Säureadditionssalzes oder Kupfer-Komplexes einer solchen Verbindung sowie Formulierungshilfsstoffe enthalten. Die Mittel enthalten zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magne siumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen

können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäurester und Butylhydroxytoluol; UV-Absorber, z.B. substituierte Benzophenone, Diphenylacrylnitrilsäureester und Zimtsäureester; und Deaktivatoren, z.B. Salze der Aethylendiamintetraessigsäure und Polyglykole.

Die erfindungsgemässen fungiziden Mittel können neben den Wirkstoffen der Formel I auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0001 und 95 Gewichtsprozent an erfindungsgemässer Verbindung bzw. erfindungsgemässen Verbindungen als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich des obigen Konzentrationsintervalls. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich des obigen Konzentrationsintervalls. Emulgierbare Konzentrate enthalten im allgemeinen 5 bis 95 Gewichtsprozent, vorzugsweise 25 bis 75 Gewichtsprozent, der Verbindung bzw. Verbindungen der Formel I. Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens

eine erfindungsgemässe Verbindung mit Formulierungshilfsstoffen vermischt.

Die Herstellung der Mittel kann in bekannter Weise durchgeführt werden, z.B. durch Vermischen der Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

## I. Herstellung der Wirkstoffe der Formel I:

### Beispiel 1

Zu einer Suspension von 73,9 g (0,215 Mol) Methoxymethyltriphenylphosphoniumchlorid in 500 ml wasserfreiem Tetrahydrofuran tropft man bei 0 °C unter Argon 135 ml n-Butyllithium (1,6m in n-Hexan) so zu, dass die Temperatur 5 °C nicht übersteigt. Nach 30-minütigem Rühren bei 0 °C wird eine Lösung von 50 g (0,178 Mol) 2-(2,4-Dichlorphenyl)-1-(3-pyridyl)-1-propanon in 100 ml trockenem Tetrahydrofuran bei -75 °C innert 30 Minuten zugetropft. Man rührt wäh rend 3 Stunden bei dieser Temperatur nach und tropft dann bei 0 °C 100 ml Wasser zu. Das Tetrahydrofuran wird unter vermindertem Druck abdestilliert und der Rückstand mit Aethylacetat extrahiert. Nach dem Abdestillieren des Aethylacetats wird der ölige Rückstand an Kieselgel mit Dichlormethan/Aethylacetat (95:5 Vol-%) als Laufmittel chromatographiert. Dabei erhält man mit der 1.Fraktion das (E)-3-(2,4-Dichlor-α-methoxymethylen-β-methylphenäthyl)-pyridin als beige Kristalle, Smp. 67-68 °C, und mit der 2.Fraktion das (Z)-3-(2,4-Dichlor-α-methoxymethylen-β-methylphenäthyl)-pyridin als gelbliches Oel im Verhältnis von ca. 1:1 in einer Gesamtmenge von 44,4 g.

In analoger Weise erhält man ausgehend von:

- 2-(2,4-Dichlorphenyl)-1-(3-pyridyl)-1-propanon und Butyltriphenylphosphoniumbromid das 3-(α-Butyliden-2,4-dichlor-β-methylphenäthyl)-pyridin.
1. Isomer: gelbes Oel
2. Isomer: gelbes Oel
- 2-(2,4-Dichlorphenyl)-1-(3-pyridyl)-1-propanon und Aethyltriphenylphosphoniumbromid das 3-(2,4-Dichlor-α-äthyliden-β-methylphenäthyl)-pyridin als farbloses Oel;
- 2-(2,4-Dichlorphenyl)-1-(3-pyridyl)-1-propanon und Chlormethyltriphenylphosphoniumchlorid das 3-(α-Chlormethylen-2,4-dichlor-β-methylphenäthyl)-pyridin als isomerenreine Verbindung in Form eines gelben Oeles;

- 2-(2,4-Dichlorphenyl)-1-(3-pyridyl)-1-propanon und Methyltriphenylphosphoniumbromid das 3-(2,4-Dichlor-β-methyl-α-methylenphenäthyl)-pyridin als hellgelbes Oel;
- 2-(2,4-Dichlorphenyl)-1-(3-pyridyl)-1-propanon und Propyltriphenylphosphoniumbromid das 3-(2,4-Dichlor-β-methyl-α-propylidenphenäthyl)-pyridin
1. Isomer: hellgelbes Oel
2. Isomer: farbloses Oel
- 2-(2,4-Dichlorphenyl)-1-(3-pyridyl)-1-butanon und Methoxymethyltriphenylphosphoniumchlorid das 3-(2,4-Dichlor-β-äthyl-α-methoxymethylenphenäthyl) pyridin als Isomerengemisch in Form eines gelben Oeles;
- 2-(2,4-Dichlorphenyl)-1-(3-pyridyl)-1-pentanon und Methyltriphenylphosphoniumbromid das 3-(2,4-Dichlor-α-methylen-β-propylphenäthyl)-pyridin als hellgelbes Oel;
- 2-(2,4-Dichlorphenyl)-1-(3-pyridyl)-4-penten-1-on und Methyltriphenylphosphoniumbromid das 3-(β-Allyl-2,4-dichlor-α-methylenphenäthyl)-pyridin als gelbes Oel;
- 2-(2,4-Dichlorphenyl)-1-(3-pyridyl)-4-pentin-1-on und Methyltriphenylphosphoniumbromid das 3-[2,4-Dichlor-α-methylen-β-(2-propinyl)-phenäthyl]-pyridin als gelbes Oel;
- 2-(2,4-Dichlorphenyl)-1-(3-pyridyl)-4-pentin-1-on und Methoxymethyltriphenylphosphoniumchlorid das 3-[2,4-Dichlor-α-methoxymethylen-β-(2-propinyl)-phenäthyl]-pyridin
1. Isomer: weisse Kristalle, Smp. 96-97° C
2. Isomer: gelbliches Oel
- 2-(2,4-Dichlorphenyl)-1-(3-pyridyl)-4-pentin-1-on und Chlormethyltriphenylphosphoniumchlorid das 3-[α-Chlormethylen-2,4-dichlor-β-(2-propinyl)-phenäthyl]-pyridin als isomerenreine Verbindung in Form weisser Kristalle, Smp. 87-88° C;
- 2-(2,4-Dichlorphenyl)-1-(3-pyridyl)-4-pentin-1-on und Butyltriphenylphosphoniumbromid das 3-[α-Butyliden-2,4-dichlor-β-(2-propinyl) -phenäthyl]-pyridin
1. Isomer: gelbes Oel
2. Isomer: gelbes Oel
- 2-(2,4-Dichlorphenyl)-2-methyl-1-(3-pyridyl)-1-propanon und Methyltriphenylphosphoniumbromid das 3-(2,4-Dichlor-β,β-dimethyl-α-methylenphenäthyl)-pyridin als gelbes Oel;
- 2′,4′-Dichlor-2-methyl-2-(3-pyridyl)-propiophenon und Methyltriphenylphosphoniumbromid das 3-(2,4-Dichlor-α,α-dimethyl-β-methylenphenäthyl)pyridin als hellgelbe Kristalle, Smp. 58-63° C;
- 2,4-Dichlorphenyl-1-(3-pyridyl)-cyclopropyl-keton und Methyltriphenylphosphoniumbromid das 3-[1-(2,4-Dichlor-α-methylenbenzyl)-cyclopropyl]-pyridin als gelbliches Oel;
- 2,4-Dichlorphenyl-1-(3-pyridyl)-cyclopropyl-keton und Methoxymethyltriphenylphosphoniumchlorid das 3-[1-(2,4-Dichlor-α-methoxymethylenbenzyl)
-cyclopropyl]-pyridin als Isomerengemisch in Form eines gelben Oeles;
- 2,4-Dichlorphenyl-1-(3-pyridyl)-cyclopropyl-keton und Aethyltriphenylphosphoniumbromid das 3-[1-(2,4-Dichlor-α-äthylidenbenzyl)-cyclopropyl]-pyridin als Isomerengemisch (ca. 10:1) in Form eines gelblichen Oeles.

## Beispiel 2

Eine Lösung von 2,5 g 3-[1-(2,4-Dichlor-α-methylenbenzyl)-cyclopropyl]-pyridin und 1,9 g 3-Chlorperbenzoesäure (85%-ig) in 30 ml Chloroform wird während 1 Stunde bei Raumtemperatur gerührt, mit 10%-iger Kaliumcarbonatlösung, dann zweimal mit Wasser gewaschen und über wasserfreien Natriumsulfat getrocknet. Das Lösungsmittel wird unter vermindertem Druck abdestilliert, wobei 2,6 g 3-[1-(2,4-Dichlor-α-methylenbenzyl)-cyclopropyl]-pyridin -1-oxid als farbloses Oel zurückbleiben.

## Beispiel 3

Lösungen von 2 g 3-(2,4-Dichlor-β,β-dimethyl-α-methylenphenäthyl)-pyridin in 80 ml Chloroform und 5 g Kupfer(II)-acetat in 30 ml Wasser werden zusammen kräftig geschüttelt. Die organische Phase wird dann über wasserfreiem Natriumsulfat getrocknet, filtriert und zur Trockene eingedampft. Nach dem Umkristallisieren des Rückstandes aus Diäthyläther/n-Hexan erhält man 1,2 g des 1:1 Komplexes von 3-(2,4-Dichlor-β,β-dimethyl-α-methylenphenäthyl)-pyridin und Kupfer(II)-acetat als grüne Kristalle, Smp. 190-192° C.

Die zur Trockene eingedampfte Mutterlauge wird in 50 ml Chloroform gelöst und nochmals in oben beschriebener Weise behandelt, wobei weitere 0,68 g Kupfer-Komplex anfallen.

In analoger Weise erhält man ausgehend von:

- 3-[1-(2,4-Dichlor-α-methylenbenzyl)-cyclopropyl]-pyridin den 1:1 Komplex von 3-[1-(2,4-Dichlor-α-methylenbenzyl)-cyclopropyl]-pyridin und Kupfer(II)-acetat als grüne Kristalle, Smp. 183° C;
- 3-(α-Butyliden2,4-dichlor-β-methylphenäthyl)-pyridin (1.Isomer) den 1:1 Komplex von 3-(α-Butyliden-2,4-dichlor-β-methylphenäthyl)-pyridin und Kupfer(II)-acetat als grüne Kristalle, Smp. 179-180° C (1.Isomer);
- 3-(2,4-Dichlor-β-methyl-α-methylenphenäthyl)-pyridin den 1:1 Komplex von 3-(2,4-Dichlor-β-methyl-α-methylenphenäthyl)-pyridin und Kupfer(II)-acetat als grüne Kristalle, Smp. 202-203° C;
- 3-(α-Butyliden-2,4-dichlor-β-methylphenäthyl)-pyridin (2.Isomer) den 1:1 Komplex von 3-(α-Butyliden-2,4-dichlor-β-methylphenäthyl)-pyridin und Kupfer(II)-acetat als grüne Kristalle, Smp. 160-161° C (2.Isomer);
- 3-(2,4-Dichlor-β-methyl-α-propylidenphenäthyl)-pyridin den 1:1 Komplex von 3-(2,4-Dichlor-β-methyl-α-propylidenphenäthyl)-pyridin und Kupfer(II)-acetat als grüne Kristalle, Smp. 191-192° C;
- 3-[2,4-Dichlor-α-methoxymethylen-β-(2-propinyl)-phenäthyl]-pyridin den 1:1 Komplex von 3- [2,4-Dichlor-α-methoxymethylen-β-(2-propinyl)-phenäthyl]-pyridin und Kupfer(II)-acetat als grüne Kristalle, Smp. 145° C.

## II. Herstellung der Ausgangsmaterialien der Formel II:

### Beispiel 4

Bei 0° bis 5° C werden zu einer Lösung von 26,6 g 2′,4′-Dichlor-2-(3-pyridyl)-acetophenon in 120 ml wasserfreiem Tetrahydrofuran unter Rühren und Inertgas portionen weise 8,72 g Natriumhydrid (55%-ig in Oel) gegeben. Nach 1,5-stündigem Rühren bei Raumtemperatur lässt man das Reaktionsgemisch auf -75° C abkühlen und bei dieser Temperatur eine Lösung von 12,45 ml Methyljodid in 20 ml Tetrahydrofuran zutropfen. Man lässt ca. 16 Stunden auf Raumtemperatur kommen, giesst auf Eiswasser und extrahiert dreimal mit je 100 ml Methylenchlorid. Nach dem Waschen mit Wasser und Abdestillieren des Lösungsmittels unter vermindertem Druck wird der Rückstand an Kieselgel mit Diäthyläther als Laufmittel chromatographiert. Man kristallisiert das Produkt aus n-Hexan und erhält dabei 15 g 2′,4′-Dichlor-2-methyl-2-(3-pyridyl)-propiophenon als beige Kristalle, Smp. 75-76° C.

In analoger Weise erhält man ausgehend von:

- 2,4-Dichlorbenzyl-(3-pyridyl)-keton das 2-(2,4-Dichlorphenyl)-2-methyl-1-(3-pyridyl)-1-propanon als hellbraune Kristalle, Smp. 103-104° C.

### Beispiel 5

Zu einer Suspension von 46,5 g frisch sublimiertem Kalium-tert.butylat in 590 ml tert.Butanol gibt man unter Inertgas portionenweise 54 g 2′,4′-Dichlor-2-(3-pyridyl)-acetophenon und rührt das Reaktionsgemisch während 2 Stunden bei 45° C. Man versetzt bei Raumtemperatur mit 59,5 g 2-Chloräthyl-dimethylsulfoniumiodid und einer Spatelspitze Natriumiodid und hält das Gemisch unter kräftigem Rühren während 16 Stunden bei 50° C. Man giesst auf Wasser und extrahiert dreimal mit je 300 ml Diäthyläther. Die vereinigten organischen Extrakte werden mit Wasser gewaschen, über wasserfreiem Natriumsulfat getrocknet und unter vermindertem Druck eingedampft. Der Rückstand wird an Kieselgel mit Diäthyläther als Laufmittel chromatographiert und aus Diäthyläther/n-Hexan kristallisiert. Man erhält 41,7 g 2,4-Dichlorphenyl-1-(3-pyridyl)-cyclopropyl-keton als weisse Kristalle, Smp. 83° C.

III. Formulierungsbeispiele:

Beispiel 6

Ein emulgierbares Konzentrat hat folgende Zusammensetzung:

| | g/Liter |
|---|---|
| Wirkstoff (erfindungsgemässe Verbindung) | 500 |
| Nonylphenolpolyäthoxylat (nicht ionischer Emulgator) | 50 |
| Calcium-dodecylbenzolsulfonat (anionischer Emulgator) | 25 |
| Gemisch von $C_{10}$-Alkylbenzolen (Lösungsmittel) | ad 1 l |

Der Wirkstoff und die Emulgatoren werden in das Lösungsmittel aufgenommen. Durch Emulgieren dieses Konzentrates kann eine gebrauchsfertige Spritzbrühe beliebiger Konzentration hergestellt werden.

Beispiel 7

Ein Saatbeizmittel hat folgende Zusammensetzung:

| | Gewichtsprozent |
|---|---|
| Wirkstoff (erfindungsgemässe Verbindung) | 10,9 |
| Natrium-laurylsulfat (Netzmittel) | 2,0 |
| Natrium-lignosulfonat (Dispergiermittel) | 4,0 |
| Dextrin (Bindemittel) | 10,0 |
| Eisenoxid-Rot (Farbstoff) | 9,0 |
| Kieselsäure (Trägerstoff) | 15,0 |
| Kaolin (Trägerstoff) | ad 100 |

Die Komponenten werden miteinander vermischt, und das Ganze wird in einer geeigneten Mühle feingemahlen. Zur Applikation kann das Mittel unmittelbar trocknen mit dem Saatgut (Samen) vermischt, oder durch Zugabe von Wasser zunächst zu einem fliessfähigen Brei übergeführt werden, der dann auf dem Saatgut verteilt werden kann.

Beispiel 8

Ein Spritzpulver hat folgende Zusammensetzung:

| | Gewichtsprozent |
|---|---|
| Wirkstoff (erfindungsgemässe Verbindung, insbesondere Kupfer-Komplex) | 25,0 |
| Natrium-laurylsulfat (Netzmittel) | 4,0 |
| Natrium-lignosulfonat (Dispergiermittel) | 10,0 |
| Kieselsäure (Trägerstoff) | 15,0 |
| Kaolin (Trägerstoff) | ad 100 |

Die Komponenten werden miteinander vermischt und das Ganze wird in einer geeigneten Mühle

feingemahlen. Durch Dispergieren des Gemisches in Wasser ergibt sich eine gebrauchsfertige Spritzbrühe.

**Ansprüche**

1. Verbindungen der allgemeinen Formel

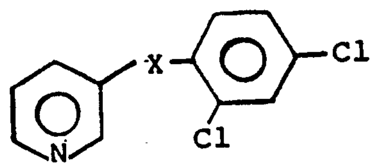

I

worin
X eine Gruppe (a) oder (b)

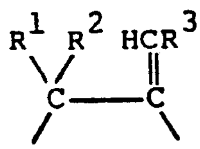

(a)

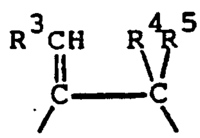

(b)

$R^1$ und $R^2$ Methyl oder zusammen Aethylen,
$R^3$ Wasserstoff, Chlor, $C_{1-4}$-Alkyl oder Methoxy,
$R^4$ Wasserstoff oder Methyl
und
$R^5$ Methyl oder, falls $R^4$ Wasserstoff bedeutet, auch $C_{1-4}$-Alkyl, Allyl oder Propargyl,
oder
$R^4$ und $R^5$ zusammen Aethylen bedeuten,
sowie die N-Oxide, N-Amino-Salze und Kupfer-Komplexe der Verbindungen der Formel I, und die Säureadditionssalze der Verbindungen der Formel I und ihrer N-Oxide.

2. Verbindungen nach Anspruch 1, worin $R^1$ und $R^2$ zusammen Aethylen bedeuten.

3. Verbindungen nach Anspruch 1 oder 2, worin $R^3$ Wasserstoff oder Methoxy bedeutet.

4. Verbindungen nach einem der Ansprüche 1 bis 3, worin $R^4$ Wasserstoff bedeutet.

5. Verbindungen nach einem der Ansprüche 1 bis 4, worin $R^5$ Methyl bedeutet.

6. Eine Verbindung nach Anspruch 1, ausgewählt aus:
3-(2,4-Dichlor-α,α-dimethyl-β-methylenphenäthyl)-pyridin,
3-[1-(2,4-Dichlor-α-methylenbenzyl)-cyclopropyl ]-pyridin,
1:1 Komplex von 3-[1-(2,4-Dichlor-α-methylenbenzyl)-cyclopropyl]-pyridin und Kupfer(II)-acetat,
3-[1-(2,4-Dichlor-α-methylenbenzyl)-cyclopropyl]-pyridin-1-oxid,
3-[1-(2,4-Dichlor-α-methoxymethylenbenzyl)-cyclopropyl]-pyridin,
3-[1-(2,4-Dichlor-α-äthylidenbenzyl)-cyclopropyl]-pyridin,
3-(2,4-Dichlor-α-methoxymethylen-β-methylphenäthyl)-pyridin,
3-(α-Butyliden-2,4-dichlor-β-methylphenäthyl)-pyridin,
3-(2,4-Dichlor-β,β-dimethyl-α-methylenphenäthyl)-pyridin,
3-[2,4-Dichlor-α-methylen-β-(2-propinyl)-phenäthyl]-pyridin,
3-[2,4-Dichlor-α-methoxymethylen-β-(2-propinyl)-phenäthyl]-pyridin,
3-(2,4-Dichlor-α-methylen-β-propylphenäthyl)-pyridin,
3-(α-Chlormethylen-2,4-dichlor-β-methylphenäthyl)-pyridin,
3-[α-Chlormethylen-2,4-dichlor-β-(2-propinyl)-phenäthyl]-pyridin,
3-(β-Allyl-2,4-dichlor-α-methylenphenäthyl)-pyridin,
1:1-Komplex von 3-(α-Butyliden-2,4-dichlor-β-methylphenäthyl)-pyridin und Kupfer(II)-acetat,

3-[α-Butyliden-2,4-dichlor-β-(2-propinyl)-phenäthyl]-pyridin,
3-(2,4-Dichlor-β-methyl-α-methylenphenäthyl)-pyridin,
1:1-Komplex von 3-(2,4-Dichlor-β-methyl-α-methylenphenäthyl)-pyridin und Kupfer(II)-acetat und
3-(2,4-Dichlor-β-äthyl-α-methoxymethylenphenäthyl)-pyridin.

7. Fungizides Mittel, dadurch gekennzeichnet, dass es eine wirksame Menge mindestens einer Verbindung der allgemeinen Formel

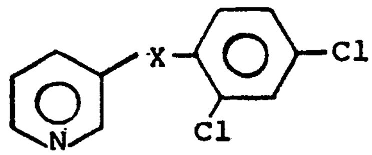

I

worin
X eine Gruppe (a) oder (b)

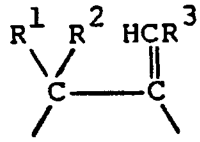

(a)

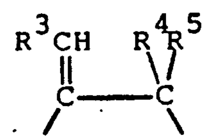

(b)

$R^1$ und $R^2$ Methyl oder zusammen Aethylen,
$R^3$ Wasserstoff, Chlor, $C_{1-4}$-Alkyl oder Methoxy,
$R^4$ Wasserstoff oder Methyl
und
$R^5$ Methyl oder, falls $R^4$ Wasserstoff bedeutet, auch $C_{1-4}$-Alkyl, Allyl oder Propargyl,
oder
$R^4$ und $R^5$ zusammen Aethylen bedeuten,
oder eines N-Oxids, N-Amino-Salzes oder Kupfer-Komplexes einer solchen Verbindung, oder eines Säureadditionssalzes einer Verbindung I oder deren N-Oxides sowie Formulierungshilfsstoffe enthält.

8. Fungizides Mittel nach Anspruch 7, dadurch gekennzeichnet, dass es eine wirksame Menge mindestens einer aus der Gruppe
3-(2,4-Dichlor-α,α-dimethyl-β-methylenphenäthyl)-pyridin,
3-[1-(2,4-Dichlor-α-methylenbenzyl)-cyclopropyl]-pyridin,
1:1 Komplex von 3-[1-(2,4-Dichlor-α-methylenbenzyl)-cyclopropyl]-pyridin und Kupfer(II)-acetat,
3-[1-(2,4-Dichlor-α-methylenbenzyl)-cyclopropyl]-pyridin-1-oxid,
3-[1-(2,4-Dichlor-α-methoxymethylenbenzyl)-cyclopropyl]-pyridin,
3-[1-(2,4-Dichlor-α-äthylidenbenzyl)-cyclopropyl]-pyridin,
3-(2,4-Dichlor-α-methoxymethylen-β-methylphenäthyl)-pyridin,
3-(α-Butyliden-2,4-dichlor-β-methylphenäthyl)-pyridin,
3-(2,4-Dichlor-β,β dimethyl-α-methylenphenäthyl)-pyridin,
3-[2,4-Dichlor-α-methylen-β-(2-propinyl)-phenäthyl]-pyridin,
3-[2,4-Dichlor-α-methoxymethylen-β-(2-propinyl)-phenäthyl]-pyridin,
3-(2,4-Dichlor-α-methylen-β-propylphenäthyl)-pyridin,
3-(α-Chlormethylen-2,4-dichlor-β-methylphenäthyl)-pyridin,
3-[α-Chlormethylen-2,4-dichlor-β-(2-propinyl)-phenäthyl]-pyridin,
3-(β-Allyl-2,4-dichlor-α-methylenphenäthyl)-pyridin,
1:1-Komplex von 3-(α-Butyliden-2,4-dichlor-β-methylphenäthyl)-pyridin und Kupfer(II)-acetat,
3-[α-Butyliden-2,4-dichlor-β-(2-propinyl)-phenäthyl]-pyridin,
3-(2,4-Dichlor-β-methyl-α-methylenphenäthyl)-pyridin,
1:1-Komplex von 3-(2,4-Dichlor-β-methyl-α-methylenphenäthyl)-pyridin und Kupfer(II)-acetat und

3-(2,4-Dichlor-β-äthyl-α-methoxymethylenphenäthyl)-pyridin ausgewählten Verbindung sowie Formulierungshilfsstoffe enthält.

9. Verfahren zur Herstellung von Verbindungen der allgemeinen Formel

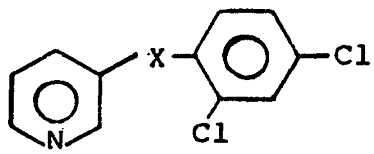

I

worin
X eine Gruppe (a) oder (b)

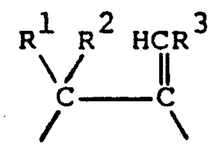

(a)

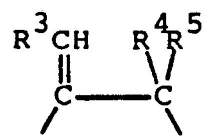

(b)

$R^1$ und $R^2$ Methyl oder zusammen Aethylen,
$R^3$ Wasserstoff, Chlor, $C_{1-4}$-Alkyl oder Methoxy,
$R^4$ Wasserstoff oder Methyl
und
$R^5$ Methyl oder, falls $R^4$ Wasserstoff bedeutet, auch $C_{1-4}$-Alkyl, Allyl oder Propargyl,
oder
$R^4$ und $R^5$ zusammen Aethylen bedeuten,
und von deren N-Oxiden, N-Amino-Salzen und Kupfer-Komplexen, bzw. Säureadditionssalzen der Verbindungen I oder deren N-Oxide, dadurch gekennzeichnet, dass man
a) ein Keton der allgemeinen Formel

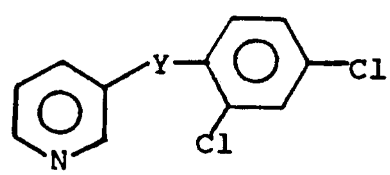

II

worin Y eine Gruppe (a′) oder (b′)

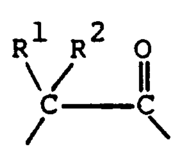

(a')

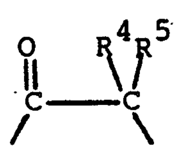

(b')

und $R^1$, $R^2$, $R^4$ und $R^5$ die oben angegebenen Bedeutungen besitzen,

mit einem Triphenylphosphoniumsalz der allgemeinen Formel
$(C_6H_5)_3P^{\oplus}CH_2R^3Hal^{\ominus}$ III
worin $R^3$ die oben angegebene Bedeutung besitzt und
$Hal^{\ominus}$ ein Chlor- oder Bromion bedeutet,
umsetzt,
b) zwecks Herstellung des N-Oxids oder eines N-Amino-Salzes einer Verbindung der Formel I diese N-oxidiert bzw. mit einem Aminderivat der allgemeinen Formel
$H_2N\text{-}A^3$ IV
worin $A^3$ den Rest einer physiologisch verträglichen Säure bedeutet,
umsetzt,
c) zwecks Herstellung eines Säureadditionssalzes einer Verbindung der Formel I oder ihres N-Oxids die Verbindung der Formel I bzw. deren N-Oxid mit einer Säure umsetzt, oder
d) zwecks Herstellung eines Kupfer-Komplexes einer Verbindung der Formel I diese mit einem Kupfersalz der allgemeinen Formel
$Cu(A^2)_2$ V
worin $A^2$ das Anion einer organischen oder anorganischen Säure bedeutet,
umsetzt.

10. Verfahren zur Bekämpfung von Fungi in der Landwirtschaft und im Gartenbau, dadurch gekennzeichnet, dass man das zu schützende Gut mit einer wirksamen Menge einer Verbindung gemäss einem der Ansprüche 1 bis 6 bzw. eines Mittels gemäss Anspruch 7 oder 8 behandelt.

11. Verwendung einer Verbindung gemäss einem der Ansprüche 1 bis 6 bzw. eines Mittels gemäss Anspruch 7 oder 8 zur Bekämpfung von Fungi in der Landwirtschaft und im Gartenbau.

Patentansprüche für folgenden Vertragsstaat: ES

1. Verfahren zur Herstellung von Verbindungen der allgemeinen Formel

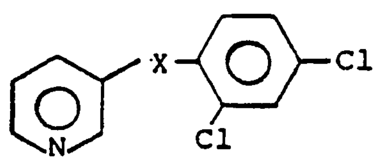

I

worin
X eine Gruppe (a) oder (b)

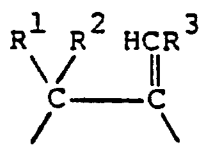

(a)

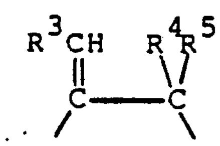

(b)

$R^1$ und $R^2$ Methyl oder zusammen Aethylen,
$R^3$ Wasserstoff, Chlor, $C_{1-4}$-Alkyl oder Methoxy,
$R^4$ Wasserstoff oder Methyl
und
$R^5$ Methyl oder, falls $R^4$ Wasserstoff bedeutet, auch $C_{1-4}$-Alkyl, Allyl Propargyl,
oder
$R^4$ und $R^5$ zusammen Aethylen bedeuten,
und von deren N-Oxiden, N-Amino-Salzen und Kupfer-Komplexen, bzw. Säureadditionssalzen der Verbindungen I oder deren N-Oxide, dadurch gekennzeichnet, dass man

a) ein Keton der allgemeinen Formel

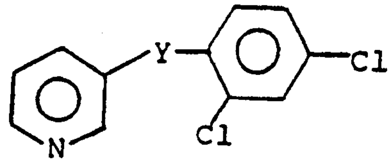

II

worin Y eine Gruppe (a′) oder (b′)

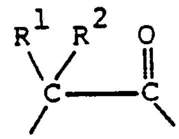

(a′)

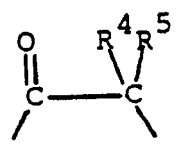

(b′)

und $R^1$, $R^2$, $R^4$ und $R^5$ die oben angegebenen Bedeutungen besitzen,
mit einem Triphenylphosphoniumsalz der allgemeinen Formel
$(C_6H_5)_3P^{\oplus}CH_2R^3Hal^{\ominus}$ III
worin $R^3$ die oben angegebene Bedeutung besitzt und Hal $^{\ominus}$ ein Chlor- oder Bromion bedeutet,
umsetzt,
b) zwecks Herstellung des N-Oxids oder eines N-Amino--Salzes einer Verbindung der Formel I diese N-oxidiert bzw. mit einem Aminderivat der allgemeinen Formel
$H_2N-A^3$ IV
worin $A^3$ den Rest einer physiologisch verträglichen Säure bedeutet,
umsetzt,
c) zwecks Herstellung eines Säureadditionssalzes einer Verbindung der Formel I oder ihres N-Oxids die Verbindung der Formel I bzw. deren N-Oxid mit einer Säure umsetzt, oder
d) zwecks Herstellung eines Kupfer-Komplexes einer Verbindung der Formel I diese mit einem Kupfersalz der allgemeinen Formel
$Cu(A^2)_2$ V
worin $A^2$ das Anion einer organischen oder anorganischen Säure bedeutet,
umsetzt.

2. Verfahren nach Anspruch 1, worin $R^1$ und $R^2$ zusammen Aethylen bedeuten.

3. Verfahren nach Anspruch 1 oder 2, worin $R^3$ Wasserstoff oder Methoxy bedeutet.

4. Verfahren nach einem der Ansprüche 1 bis 3, worin $R^4$ Wasserstoff bedeutet.

5. Verfahren nach einem der Ansprüche 1 bis 4, worin $R^5$ Methyl bedeutet.

6. Verfahren nach Anspruch 1 zur Herstellung einer aus der Gruppe
3-(2,4-Dichlor-α,α-dimethyl-β-methylenphenäthyl)-pyridin,
3-[1-(2,4-Dichlor-α-methylenbenzyl)-cyclopropyl]-pyridin,
1:1 Komplex von 3-[1-(2,4-Dichlor-α-methylenbenzyl)-cyclopropyl]-pyridin und Kupfer(II)-acetat,
3-[1-(2,4-Dichlor-α-methylenbenzyl)-cyclopropyl]-pyridin-1-oxid,
3-[1-(2,4-Dichlor-α-methoxymethylenbenzyl)-cyclopropyl]-pyridin,
3-[1-(2,4-Dichlor-α-äthylidenbenzyl)-cyclopropyl]-pyridin,
3-(2,4-Dichlor-α-methoxymethylen-β-methylphenäthyl)-pyridin,
3-(α-Butyliden-2,4-dichlor-β-methylphenäthyl)-pyridin,
3-(2,4-Dichlor-β,β-dimethyl-α-methylenphenäthyl)-pyridin,
3-[2,4-Dichlor-α-methylen-β-(2-propinyl)-phenäthyl]-pyridin,
3-[2,4-Dichlor-α-methoxymethylen-β-(2-propinyl)-phen äthyl]-pyridin,
3-(2,4-Dichlor-α-methylen-β-propylphenäthyl)-pyridin,
3-(α-Chlormethylen-2,4-dichlor-β-methylphenäthyl)-pyridin,

3-[α-Chlormethylen-2,4-dichlor-β-(2-propinyl)-phenäthyl]-pyridin,
3-(β-Allyl-2,4-dichlor-α-methylenphenäthyl)-pyridin,
1:1-Komplex von 3-(α-Butyliden-2,4-dichlor-β-methylphenäthyl)-pyridin und Kupfer(II)-acetat,
3-[α-Butyliden-2,4-dichlor-β-(2-propinyl)-phenäthyl]-pyridin,
3-(2,4-Dichlor-β-methyl-α-methylenphenäthyl)-pyridin,
1:1-Komplex von 3-(2,4-Dichlor-β-methyl-α-methylenphenäthyl)-pyridin und Kupfer(II)-acetat und
3-(2,4-Dichlor-β-äthyl-α-methoxymethylenphenäthyl)pyridin
ausgewählten Verbindung.

7. Fungizides Mittel, dadurch gekennzeichnet, dass es eine wirksame Menge mindestens einer Verbindung der allgemeinen Formel

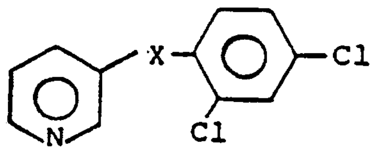

I

worin
X eine Gruppe (a) oder (b)

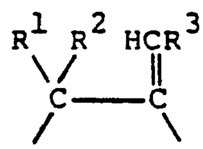

(a)

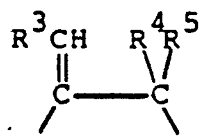

(b)

$R^1$ und $R^2$ Methyl oder zusammen Aethylen,
$R^3$ Wasserstoff, Chlor, $C_{1-4}$-Alkyl oder Methoxy,
$R^4$ Wasserstoff oder Methyl
und
$R^5$ Methyl oder, falls $R^4$ Wasserstoff bedeutet, auch $C_{1-4}$-Alkyl, Allyl oder Propargyl,
oder
$R^4$ und $R^5$ zusammen Aethylen bedeuten,
oder eines N-Oxids, N-Amino-Salzes oder Kupfer-Komplexes einer solchen Verbindung, oder eines Säureadditionssalzes einer Verbindung 1 oder deren N-Oxides sowie Formulierungshilfsstoffe enthält.

8. Fungizides Mittel nach Anspruch 7, dadurch gekennzeichnet, dass es eine wirksame Menge mindestens einer aus der Gruppe
3-(2,4-Dichlor-α,α-dimethyl-β-methylenphenäthyl)-pyridin,
3-[1-(2,4-Dichlor-α-methylenbenzyl)-cyclopropyl]-pyridin,
1:1 Komplex von 3-[1-(2,4-Dichlor-α-methylenbenzyl)-cyclopropyl]-pyridin und Kupfer(II)-acetat,
3-[1-(2,4-Dichlor-α-methylenbenzyl)-cyclopropyl]-pyridin-1-oxid,
3-[1-(2,4-Dichlor-α-methoxymethylenbenzyl)-cyclopropyl]-pyridin,
3-[1-(2,4-Dichlor-α-äthylidenbenzyl)-cyclopropyl]-pyridin,
3-(2,4-Dichlor-α-methoxymethylen-β-methylphenäthyl)-pyridin,
3-(α-Butyliden-2,4-dichlor-β-methylphenäthyl)-pyridin,
3-(2,4-Dichlor-β,β-dimethyl-α-methylenphenäthyl)-pyridin,
3-[2,4-Dichlor-α-methylen-β-(2-propinyl)-phenäthyl]-pyridin,
3-[2,4-Dichlor-α-methoxymethylen-β-(2-propinyl)-phenäthyl]-pyridin,
3-(2,4-Dichlor-α-methylen-β-propylphenäthyl)-pyridin,
3-(α-Chlormethylen-2,4-dichlor-β-methylphenäthyl)-pyridin,
3-[α-Chlormethylen-2,4-dichlor-β-(2-propinyl)-phenäthyl]-pyridin,

3-(β-Allyl-2,4-dichlor-α-methylenphenäthyl)-pyridin,
1:1-Komplex von 3-(α-Butyliden-2,4-dichlor-β-methylphenäthyl)-pyridin und Kupfer(II)-acetat,
3-[α-Butyliden-2,4-dichlor-β-(2-propinyl)-phenäthyl]-pyridin,
3-(2,4-Dichlor-β-methyl-α-methylenphäthyl)-pyridin,
1:1-Komplex von 3-(2,4-Dichlor-β-methyl-α-methylenphenäthyl)-pyridin und Kupfer(II)-acetat und
3-(2,4-Dichlor-β-äthyl-α-methoxymethylenphenäthyl)-pyridin
ausgewählten Verbindung sowie Formulierungshilfsstoffe enthält.

9. Verfahren zur Herstellung eines fungiziden Mittels gemäss Anspruch 7, dadurch gekennzeichnet, dass man mindestens eine Verbindung der allgemeinen Formel I mit Formulierungshilfsstoffen vermischt.